# FASCICULE DE BREVET EUROPEEN

(11) **EP 1 145 706 B1**
(45) Date de publication et mention de la délivrance du brevet: **28.06.2017**
(21) Numéro de dépôt: 01400780.1
(22) Date de dépôt: 27.03.2001
(51) Int. Cl.: A61K 8/67, A61Q 19/08

(54) **Composition, notamment cosmétique, comprenant de l'acide acorbique en association avec un dérivé d'acide ascorbique.**
Mittel insbesondere für Kosmetika enthaltend Ascorbinsäure in Kombination mit einem Ascorbinsäurederivat
Composition particularly for cosmetics comprising ascorbic acid in combination with an ascorbic acid derivative

(30) Priorité: 10.04.2000 FR 0004575
(43) Date de publication de la demande: 17.10.2001
(73) Titulaire: L'Oréal, 75008 Paris (FR)
(72) Inventeur: Castiel, Isabelle, 78350 Jouy-en-Josas (FR); Ferraris, Corinne, 75017 Paris (FR); Bouchard, Arnelle, 18240 Boulleret (FR)
(74) Mandataire: Nony

(56) Documents cités:
- EP-A- 0 487 404
- US-A- 5 470 874
- US-A- 5 607 921
- US-A- 5 801 192
- PATENT ABSTRACTS OF JAPAN vol. 011, no. 124 (C-416), 17 avril 1987 (1987-04-17) & JP 61 263906 A (SHISEIDO CO LTD), 21 novembre 1986 (1986-11-21)
- PATENT ABSTRACTS OF JAPAN vol. 1997, no. 04, 30 avril 1997 (1997-04-30) & JP 08 333260 A (KAMINOMOTO HONPO:KK), 17 décembre 1996 (1996-12-17)
- PATENT ABSTRACTS OF JAPAN vol. 017, no. 659 (C-1137), 7 décembre 1993 (1993-12-07) & JP 05 213736 A (UNITIKA LTD), 24 août 1993 (1993-08-24)
- PATENT ABSTRACTS OF JAPAN vol. 1996, no. 02, 29 février 1996 (1996-02-29) & JP 07 252127 A (TSUMURA & CO), 3 octobre 1995 (1995-10-03)
- CHEMICAL ABSTRACTS, vol. 125, no. 25, 16 décembre 1996 (1996-12-16) Columbus, Ohio, US; abstract no. 326763f, TAKAYUKI WATANABE ET AL: "Stability of L-ascorbyl-2-O-alpha-glucoside and L-ascorbyl-2-phosphate Mg" page 1223; XP002157521 & SUISAN ZOSHOKU, vol. 44, no. 3, 1996, pages 369-373,

## Description

L'invention décrit une association comprenant de l'acide ascorbique et au moins un composé choisi parmi : un ester d'ose de l'acide ascorbique et un sel métallique d'acide ascorbique phosphorylé, ainsi que son utilisation pour augmenter la synthèse des céramides épidermiques. Elle concerne également une composition comprenant cette association dans un milieu physiologiquement acceptable, ainsi que ses utilisations.

La peau humaine est constituée de deux compartiments à savoir un compartiment profond, le derme, et un compartiment superficiel, l'épiderme.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. Il contient également des vaisseaux sanguins et des fibres nerveuses.

L'épiderme est en contact avec l'environnement extérieur. Son rôle consiste à protéger l'organisme de la déshydratation et des agressions extérieures, qu'elles soient chimiques, mécaniques, physiques ou infectieuses.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Les cellules constituant l'épiderme sont délimitées par un domaine lipidique. Au cours de la différenciation, les phospholipides dont le rôle consiste à élaborer la structure fluide des membranes cellulaires des couches vivantes de l'épiderme, sont peu à peu remplacés par un mélange composé en majeure partie d'acides gras, de cholestérol et de sphingolipides.

Ces lipides sont organisés en structures lamellaires spécifiques dont l'intégrité dépend non seulement de la qualité des fractions présentes mais aussi de leur proportion respective.

Cette structure lamellaire des lipides du domaine lipidique de l'épiderme est responsable de la fluidité donc de la souplesse de la peau.

Les lipides sont également responsables des propriétés "barrière" de l'épiderme particulièrement du stratum comeum.

Les lipides épidermiques sont synthétisés principalement dans l'épiderme vivant. Ils sont principalement constitués de phospholipides, de sphingolipides, de cholestérol, d'acides gras libres, de triglycérides, d'esters du cholestérol et d'alcanes.

Les phospholipides sont essentiels pour la constitution des membranes cellulaires. Ils jouent un rôle important dans la médiation des signaux extracellulaires et la formation de chaînes aliphatiques libres utilisées pour la production d'énergie. Ils constituent un réservoir d'acides gras libres nécessaires à la constitution des sphingolipides.

Les sphingolipides (ou céramides) sont essentiels pour le maintien de la structure multilamellaire des lipides intercornéocytaires. Ils sont également essentiels pour les échanges en eau et la fonction "barrière" de l'épiderme.

Le cholestérol joue un rôle primordial dans l'hydratation cutanée et dans la fonction "barrière" de l'épiderme.

Les acides gras libres jouent un rôle majeur dans le maintien de la structure lamellaire des lipides du stratum corneum, ainsi que dans la constitution des membranes cellulaires où ils sont responsables de la fluidité membranaire mais également de processus physiologiques tels que le fonctionnement de récepteurs ou l'activité enzymatique.

On comprend alors le rôle essentiel que jouent les lipides de la peau et l'importance que revêt leur intégrité.

Or, dans certaines situations, qu'il s'agisse de pathologies spécifiques (dermite atopique), de vieillissement cutané, de vieillissement actinique, de peau sèche ou encore de fonction barrière altérée par des agressions répétées, physiques ou chimiques, l'épiderme humain présente des modifications de sa composition et/ou de sa synthèse lipidique.

Afin d'améliorer le contenu en lipides de l'épiderme et par conséquent d'agir sur la souplesse de la peau, deux voies d'action sont envisageables. La première est l'apport exogène de composés lipidiques par voie topique. La deuxième consiste à stimuler la synthèse des lipides endogènes. Il a ainsi été démontré qu'il était possible d'améliorer le profil lipidique des épidermes reconstruits en ajoutant de l'acide ascorbique (vitamine C) dans le milieu de culture (J. Invest. Dermatol. 109 :348-355, 1997). Toutefois, en raison de sa structure chimique (d'alpha-cétolactone), l'acide ascorbique est très sensible à certains paramètres de l'environnement comme la lumière, la chaleur et les milieux aqueux, en particulier les milieux alcalins et/ou aérobies. En raison de ces problèmes de stabilité, il est nécessaire d'utiliser de fortes concentrations d'acide ascorbique pour observer l'effet sur la peau d'une composition le contenant.

Il est connu du document JP61263906 la mise en oeuvre d'une association d'acide ascorbique avec un tensioactif à des fins de dépigmentation de la peau.

Il est en outre décrit dans le brevet US5,470,874 la mise en oeuvre d'une composition comprenant de l'acide ascorbique, notamment en association avec une proanthocyanidine, à des fins de réparation et de stimulation de la croissance de la peau humaine.

Le brevet US5.801.192 décrit quant à lui la mise en oeuvre d'une composition comprenant de l'acide ascorbique, ou certains de ces dérivés, afin de stimuler la production d'élastine au niveau du derme.

Le brevet US5.607.921 vise à améliorer la stabilité de compositions cosmétiques ou dermatologiques comprenant des actifs instables, parmi lesquels les vitamines, tel que l'acide ascorbique.

Wu et al., Growth Horm IGF Res. 1998 Oct:8(5):421-8. décrit la mise en oeuvre individuelle de l'acide ascorbique et de ses dérives pour stimuler la synthèse d'un facteur de croissance des hépatocytes impliqué dans la dégénération des organes, la cicatrisation et l'embryogenèse.

Le brevet EP0487404 décrit une composition cosmétique stable comprenant de l'acide ascorbique, la composition ayant pour effets d'embellir la peau, de la blanchir et de prévenir la peau rugueuse.

Le document EP0998914 décrit quant à lui des compositions comprenant un actif hydrophobe ou hydrophile et au moins un émulsifiant polymérique améliorant la pénétration de l'actif dans la peau, sans augmenter son caractère irritant, l'agent hydrophobe ou hydrophile pouvant être l'acide ascorbique ou l'un de ses dérivés.

La Demanderesse a maintenant découvert, de manière surprenante, que l'association d'au moins un dérivé donné d'acide ascorbique à l'acide ascorbique lui-même entraînait une synergie d'action sur la lipogénèse de l'épiderme et précisément sur la synthèse des céramides épidermiques. Cette synergie permet l'utilisation d'une moindre quantitié d'acide ascorbique, au profit de dérivés plus stables et donc plus faciles à formuler dans des compositions cosmétiques ou dermatologiques.

L'invention décrit une association comprenant de l'acide ascorbique et au moins un composé choisi parmi : un ester d'ose de l'acide ascorbique et un sel métallique d'acide ascorbique phosphorylé.

Les esters d'ose de l'acide ascorbique utilisables sont notamment les dérivés glycosylé, mannosylé, fructosylé, fucosylé, galactosylé N-acétylglucosaminé, N-acétylmuramique de l'acide ascorbique et leurs mélanges et plus spécialement l'ascorbyl-2 glucoside ou 2-O-α-D glucopyranosyl de l'acide L-ascorbique ou encore le 6-O-β-D galactopyranosyl de l'acide L-ascorbique. Ces derniers composés ainsi que leurs procédés de préparation, sont en particulier décrits dans les documents EP-A-487404, EP-A-425066 et J05213736.

De son côté, le sel métallique d'acide ascorbique phosphorylé est choisi parmi les ascorbyl phosphates de métal alcalin, les ascorbyl phosphates de métal alcalino-terreux et les ascorbyl phosphates de métal de transition. On utilise avantageusement l'ascorbyl phosphate de magnésium.

Selon une forme de réalisation préférée, l'association selon l'invention comprend de l'acide ascorbique, un ester d'ose de l'acide ascorbique et un sel métallique d'acide ascorbique phosphorylé.

L'invention décrit également l'utilisation de l'association ci-dessus pour fabriquer une composition destinée à augmenter la synthèse des céramides épidermiques.

Par "céramides épidermiques", on entend, selon la présente description, aussi bien les céramides de type I à VII, en particulier les céramides de type IV à VII, que les acylglucosylcéramides.

L'invention a pour objet une composition cosmétique ou dermatologique comprenant, dans un milieu physiologiquement acceptable, une association comprenant de l'acide ascorbique et un sel métallique d'acide ascorbique phosphorylé, le composition. comprenant de 0.0001 à 15% en poids d'acide ascorbique et de 0.001 à 10% en poids de sel métallique d'acide ascorbique phosphorylé, par rapport au poids total de la composition.

Compte tenu des propriétés de l'association précitée sur la lipogénèse, cette composition est avantageusement utilisée pour fabriquer une préparation destinée à améliorer la fonction barrière de la peau. Une telle préparation peut être destinée au traitement de certaines pathologies impliquant une perturbation de la fonction barrière, telles que la dermite atopique ou séborrhéique. Elle peut en variante être utilisée à des fins cosmétiques, en particulier pour améliorer la souplesse de la peau et/ou l'aspect de surface de la peau et/ou lutter contre ou prévenir le vieillissement de la peau.

L'amélioration de la fonction barrière conduisant à une meilleure rétention d'eau dans la peau, la composition selon l'invention peut également être utilisée dans un procédé cosmétique d'hydratation de la peau, comprenant l'application sur la peau de cette composition.

Dans toutes les applications ci-dessus, la composition selon l'invention renferme de 0,0001 à 15% en poids, et plus préférentiellement de 0,5 à 5% en poids, d'acide ascorbique, par rapport au poids total de la composition ; de 0,001 à 10% en poids, et plus préférentiellement de 0,01 à 0,5% en poids, d'ester d'ose de l'acide ascorbique, par rapport au poids total de la composition ; et de 0,001 à 10% en poids, plus préférentiellement de 0,01 à 0,5% en poids, de sel métallique d'acide ascorbique phosphorylé, par rapport au poids total de la composition.

La composition de l'invention peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, en particulier sous forme de stick pour les lèvres lorsqu'elle est par exemple destinée à traiter les fissures labiales. Elle peut être utilisée comme produit de soin du visage ou du corps et/ou comme produit de maquillage pour la peau.

De façon connue, la composition de l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse, dans les vésicules lipidiques et/ou dans les nanoparticules. En tout état de cause, ces adjuvants, ainsi que leurs proportions, seront choisis de manière à ne pas nuire aux propriétés recherchées de l'association d'actifs selon l'invention.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-20, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les agents kératolytiques et/ou desquamants, les agents dépigmentants, les filtres UV, les agents anti-radicaux libres et leurs mélanges. En cas d'incompatibilité, certains au moins des actifs peuvent être incorporés dans des sphérules, notamment des vésicules ioniques ou non-ioniques et/ou des nanoparticules (nanocapsules et/ou nanosphères), de manière à ce que les actifs incompatibles entre eux soient isolés les uns des autres dans la composition.

Selon une autre forme d'exécution de l'invention, la composition précitée peut être utilisée améliorer le contenu en lipides et/ou la fonction barrière d'un épiderme reconstruit. L'addition conjointe d'acide ascorbique et de l'un au moins de ses dérivés dans le milieu de culture des épidermes reconstruits permet ainsi de rapprocher ces épidermes de la structure de la peau humaine normale et, par là même, de rendre les tests in vitro (notamment les études de pénétration) réalisés sur ces épidermes plus prédictifs des phénomènes qui seront observés in vivo.

Dans ce cas, la composition selon l'invention renferme de préférence de 0,0000001 à 0,1% en poids, et plus préférentiellement de 0,0005 à 0,05% en poids, d'acide ascorbique, par rapport au poids total de la composition ; de 0,0000001 à 0,01% en poids, et plus préférentiellement de 0,000005 à 0,005% en poids, d'ester d'ose de l'acide ascorbique, par rapport au poids total de la composition ; et de 0,0000001 à 0,01% en poids, plus préférentiellement de 0,000005 à 0,005% en poids, de sel métallique d'acide ascorbique phosphorylé, par rapport au poids total de la composition.

L'invention sera mieux comprise, et ses avantages ressortiront mieux, à la lumière des exemples suivants, qui sont donnés à titre illustratif, et sans limitation.

### Exemple 1 : Mise en évidence de la synergie d'action du mélange des vitamines C, CP et CG sur la synthèse des céramides épidermiques

L'acide ascorbique et ses dérivés sont testés sur un équivalent de peau vendu par la société EPISKIN (LYON, France), après culture de celui-ci pendant 7 jours. Les milieux de culture et d'essais sont ceux inclus dans le kit vendu par le fournisseur. Chaque composé a été testé individuellement à 15 µg/ml et, en association, à 5 µg/ml (en équivalent vitamine C), dans le milieu de culture. Les épidermes reconstruits sont traités durant 24 heures. Le témoin est constitué par un équivalent d'épiderme identique subissant une application topique de milieu de culture sans composé à tester.

L'épiderme reconstruit est incubé pendant une nuit avec de l'acétate ¹⁴C (2 µCi/ml) avant application topique de la vitamine C et de ses dérivés, pour suivre la synthèse des lipides.

A la fin de l'incubation, on détache l'équivalent d'épiderme de son support collagénique. La préparation des lipides de l'équivalent d'épiderme et leur analyse par HPTLC ou chromatographie sur couche mince haute performance, sont réalisées selon la technique et avec les tampons décrits par M. Ponec (1991, Adv. Lipid Res. 24:83-117).

En fin de migration, l'analyse densitométrique de l'autoradiographie est réalisée à l'aide d'un densitomètre de marque FUJI, modèle 1800.

Le tableau 1 ci-dessous rassemble les résultats obtenus, pour les trois dérivés de vitamine C testés chacun à 15 µg/ml, en pourcentage d'augmentation des céramides par rapport au témoin non traité.

**TABLEAU 1**

| Composé testé | % d'augmentation des céramides dans l'équivalent d'épiderme |
|---|---|
| Vitamine C | 54% |
| Vitamine CP | 99% |
| Vitamine CG | 69% |

La valeur (cumulée) théorique du mélange des trois dérivés de vitamine C à 5 µg/ml est donc de : (54 + 99 + 69) 13 = 74.

Or, la valeur réellement obtenue pour le mélange des trois dérivés de vitamine C, testés à 5 µg/ml chacun, est de 114.

Cet exemple démontre donc clairement qu'après seulement 24h de traitement, l'association des différents types de vitamines C entraîne une synergie d'action sur la synthèse des céramides épidermiques.

### Exemple 2 : Mise en évidence de la synergie d'action du mélange des vitamines C et CP sur la synthèse des céramides épidermiques.

On utilise le mode opératoire décrit dans l'Exemple 1, excepté que l'essai est effectué sur 48 heures au lieu de 24 heures. En outre, seules la vitamine C et la vitamine CP sont testées, le mélange des deux étant réalisé en utilisant 7,5 µg/ml de chaque vitamine.

Le tableau 2 ci-dessous rassemble les résultats obtenus, pour les deux dérivés de vitamine C testés chacun à 15 µg/ml, en pourcentage d'augmentation des céramides par rapport au témoin non traité.

**TABLEAU 2**

| Composé testé | % d'augmentation des céramides dans l'équivalent d'épiderme |
|---|---|
| Vitamine C | 126% |
| Vitamine CP | 148% |

La valeur (cumulée) théorique du mélange des deux dérivés de vitamine C à 7,5 µg/ml est donc de : (126 + 148) / 2 =137.

Or, la valeur réellement obtenue pour le mélange des deux dérivés de vitamine C, testés à 7,5 µg/ml chacun, est de 170.

Cet exemple démontre donc clairement une action synergique de l'association vitamine C et vitamine CP sur la synthèse des céramides épidermiques, après 48h de traitement seulement.

### Exemple 3 : Composition cosmétique

On a préparé la composition suivante de façon classique :

| | |
|---|---|
| Octyldodécanol | 0,2 % |
| Cyclométhicone | 5 % |
| Diméthicone copolyol | 5 % |
| Acétate de tocophéryle | 1 % |
| Filtre UV | 1 % |
| Acide ascorbique | 0,01 % |
| Ascorbyl phosphate de magnésium | 0,1 % |
| Glucoside d'ascorbyle | 0,1 % |
| Glycérine | 3 % |
| EDTA disodique | 0,1 % |
| Ajusteurs de pH | 2,6 % |
| Conservateurs | 0,4 % |
| Gélifiants | 1,2 % |
| Eau | qsp. 100 % |

On obtient un fluide qui peut être appliqué matin et/ou soir sur le visage pour améliorer la souplesse de la peau et lisser les rides et ridules.

## Revendications

1. Composition cosmétique ou dermatologique comprenant, dans un milieu physiologiquement acceptable, une association comprenant au moins de l'acide ascorbique et un sel métallique d'acide ascorbique phosphorylé, la composition comprenant de 0,0001 à 15 % en poids d'acide ascorbique et de 0,001 à 10% en poids de sel métallique d'acide ascorbique phosphorylé, par rapport au poids total de la composition.

2. Composition selon la revendication 1, **caractérisée en ce qu'**elle comprend en outre un composé choisi parmi le 2-O-α-D glucopyranosyl de l'acide L-ascorbique et le 6-O-β-D galactopyranosyl de l'acide L-ascorbique, ce composé étant présent dans une teneur allant de 0,001 à 10% en poids, par rapport au poids total de la composition.

3. Composition selon l'une des revendications 1 ou 2, **caractérisée en ce que** le sel métallique d'acide ascorbique phosphorylé est choisi parmi les ascorbyl phosphates de métal alcalin, les ascorbyl phosphates de métal alcalino-terreux et les ascorbyl phosphates de métal de transition.

4. Composition selon l'une quelconque des revendications 1 à 3, **caractérisée en ce que** ledit sel métallique d'acide ascorbique phosphorylé est l'ascorbyl phosphate de magnésium.

5. Composition selon l'une des revendications précédentes, **caractérisée en ce qu'**elle renferme de 0,0001 à 5% en poids d'acide ascorbique, par rapport au poids total de la composition.

6. Composition selon l'une quelconque des revendications 2 à 4, **caractérisée en ce qu'**elle renferme de 0,01 à 0,5% en poids d'ester d'ose de l'acide ascorbique, par rapport au poids total de la composition.

7. Composition selon l'une quelconque des revendications 1 à 4, **caractérisée en ce qu'**elle renferme de 0,01 à 0,5% en poids de sel métallique d'acide ascorbique phosphorylé, par rapport au poids total de la composition.

8. Utilisation d'une composition cosmétique selon l'une quelconque des revendications 1 à 7 pour améliorer la fonction barrière de la peau.

9. Composition telle que définie dans l'une quelconque des revendications 1 à 7 pour le traitement de la dermite atopique ou séborrhéique.

10. Utilisation d'une composition cosmétique selon l'une quelconque des revendications 1 à 7 pour améliorer la souplesse de la peau et/ou l'aspect de surface de la peau et/ou lutter contre ou prévenir le vieillissement de la peau.

11. Procédé cosmétique d'hydratation de la peau, comprenant l'application sur la peau d'une composition selon l'une quelconque des revendications 1 à 7.

12. Utilisation d'une composition cosmétique comprenant, dans un milieu physiologiquement acceptable, une association comprenant de l'acide ascorbique, au moins un composé choisi parmi le 2-O-α-D glucopyranosyl de l'acide L-ascorbique et le 6-O-β-D galactopyranosyl de l'acide L-ascorbique et un sel métallique d'acide ascorbique phosphorylé pour améliorer *in vitro* le contenu en lipides et/ou la fonction barrière d'un épiderme reconstruit, la composition renfermant de 0,0005 à 0,05% en poids d'acide ascorbique, de 0,000005 à 0,005% en poids de 2-O-α-D glucopyranosyl de l'acide L-ascorbique ou de 6-O-β-D galactopyranosyl de l'acide L-ascorbique et de 0,000005 à 0,005% en poids de sel métallique d'acide ascorbique phosphorylé, par rapport au poids total de la composition.

13. Utilisation selon la revendication précédente, **caractérisée en ce que** ledit sel métallique d'acide ascorbique phosphorylé est tel que définit dans l'une des revendications 3 ou 4.

## Patentansprüche

1. Kosmetische oder dermatologische Zusammensetzung umfassend, in einem physiologisch verträglichen Medium, eine Kombination umfassend mindestens Ascorbinsäure und ein Metallsalz von phosphorylierter Ascorbinsäure, wobei die Zusammensetzung von 0,0001 bis 15 Gew.-% Ascorbinsäure und von 0,001 bis 10 Gew.-% Metallsalz von phosphorylierter Ascorbinsäure bezogen auf das Gesamtgewicht der Zusammensetzung umfasst.

2. Zusammensetzung nach Anspruch 1, **dadurch gekennzeichnet, dass** sie weiterhin eine Verbindung umfasst, die ausgewählt ist aus 2-O-α-D-Glucopyranosyl-L-ascorbinsäure und 6-O-ß-D-Galactopyranosyl-L-ascorbinsäure, wobei diese Verbindung in einem Gehalt von 0,001 bis 10 Gew.-%, bezogen auf das Gesamtgewicht der Zusammensetzung vorhanden ist.

3. Zusammensetzung nach Anspruch 1 oder 2, **dadurch gekennzeichnet, dass** das Metallsalz von phosphorylierter Ascorbinsäure ausgewählt ist aus Alkalimetall-Ascorbylphosphaten, Erdalkalimetall-Ascorbylphosphaten und Übergangsmetall-Ascorbylphosphaten.

4. Zusammensetzung nach einem der Ansprüche 1 bis 3, **dadurch gekennzeichnet, dass** das Metallsalz von phosphorylierter Ascorbinsäure Magnesiumascorbylphosphat ist.

5. Zusammensetzung nach einem der vorangehenden Ansprüche, **dadurch gekennzeichnet, dass** sie 0,0001 bis 5 Gew.-% Ascorbinsäure bezogen auf das Gesamtgewicht der Zusammensetzung umfasst.

6. Zusammensetzung nach einem der Ansprüche 2 bis 4, **dadurch gekennzeichnet, dass** sie von 0,01 bis 0,5 Gew.-% Ascorbinsäure-Ose-Ester bezogen auf das Gesamtgewicht der Zusammensetzung umfasst.

7. Zusammensetzung nach einem der Ansprüche 1 bis 4, **dadurch gekennzeichnet, dass** sie von 0,01 bis 0,5 Gew.-% Metallsalz von phosphorylierter Ascorbinsäure bezogen auf das Gesamtgewicht der Zusammensetzung umfasst.

8. Verwendung einer kosmetischen Zusammensetzung nach einem der Ansprüche 1 bis 7 zur Verbesserung der Barrierefunktion der Haut.

9. Zusammensetzung nach einem der Ansprüche 1 bis 7 zur Behandlung atopischer oder seborrhoischer Dermatitis.

10. Verwendung einer kosmetischen Zusammensetzung nach einem der Ansprüche 1 bis 7 zur Verbesserung der Elastizität der Haut und/oder des Oberflächenaussehens der Haut und/oder zur Bekämpfung oder Vorbeugung der Alterung der Haut.

11. Kosmetisches Hydratationsverfahren der Haut, umfassend das Aufbringen einer Zusammensetzung nach einem der Ansprüche 1 bis 7 auf die Haut.

12. Verwendung einer kosmetischen Zusammensetzung umfassend, in einem physiologisch verträglichen Medium, eine Kombination umfassend Ascorbinsäure, mindestens eine Verbindung ausgewählt aus 2-O-α-D-Glucopyranosyl-L-ascorbinsäure und 6-O-ß-D-Galactopyranosyl-L-ascorbinsäure und einem Metallsalz von phosphorylierter Ascorbinsäure zur Verbesserung des Gehalts an Lipiden in vitro und/oder der Barrierefunktion einer rekonstruierten Epidermis, wobei die Zusammensetzung von 0,0005 bis 0,05 Gew.-% Ascorbinsäure, von 0,000005 bis 0,005 Gew.-% 2-O-α-D-Glucopyranosyl-L-ascorbinsäure oder 6-O-ß-D-Galactopyranosyl-L-ascorbinsäure und von 0,000005 bis 0,005 Gew.-% von phosphorylierter Ascorbinsäure bezogen auf das Gesamtgewicht der Zusammensetzung umfasst.

13. Verwendung nach dem vorangehenden Anspruch, **dadurch gekennzeichnet, dass** das Metallsalz von phosphorylierter Ascorbinsäure wie in einem der Ansprüche 3 oder 4 ist.

## Claims

1. Cosmetic or dermatological composition comprising, in a physiologically acceptable medium, an association comprising at least ascorbic acid and a metal salt of phosphorylated ascorbic acid, the composition comprising from 0.0001 to 15 % by weight of ascorbic acid and from 0.001 to 10 % by weight of metal salt of phosphorylated ascorbic acid relative to the total weight of the composition.

2. The composition according to claim 1, **characterized in that** it further comprises a compound selected from among 2-O-α-D glucopyranosyl of L-ascorbic acid and 6-O-β-D galactopyranosyl of L-ascorbic acid, this compound being contained in an amount ranging from 0.001 to 10 % by weight relative to the total weight of the composition.

3. The composition according to one of claims 1 or 2, **characterized in that** the metal salt of phosphorylated ascorbic acid is selected from among ascorbyl phosphates of alkaline metals, ascorbyl phosphates of alkaline-earth metals and ascorbyl phosphates of transition metals.

4. The composition according to any of claims 1 to 3, **characterized in that** said metal salt of phosphorylated ascorbic acid is magnesium ascorbyl phosphate.

5. The composition according to one of the preceding claims, **characterized in that** it contains from 0.0001 to 5 % by weight of ascorbic acid relative to the total weight of the composition.

6. The composition according to any of claims 2 to 4, **characterized in that** it contains from 0.01 to 0.5 % by weight of an ose ester of ascorbic acid relative to the total weight of the composition.

7. The composition according to any of claims 1 to 4, **characterized in that** it contains from 0.01 to 0.5 % by weight of metal salt of phosphorylated ascorbic acid relative to the total weight of the composition.

8. Use of a cosmetic composition according to any of claims 1 to 7 to improve the barrier function of the skin.

9. A composition such as defined in any of claims 1 to 7 for the treatment of atopic or seborrheic dermatitis.

10. Use of a cosmetic composition according to any of claims 1 to 7 to improve skin suppleness and/or skin surface appearance and/or to combat or prevent ageing of the skin.

11. A cosmetic method to hydrate the skin comprising the application to the skin of a composition according to any of claims 1 to 7.

12. Use of a cosmetic composition comprising, in a physiologically acceptable medium, an association comprising ascorbic acid, at least one compound selected from among 2-o-α-D glucopyranosyl of L-ascorbic acid and 6-O-β-D galactopyranosyl of L-ascorbic acid and a metal salt of phosphorylated ascorbic acid for *in vitro* improvement of the lipid content and/or barrier function of a reconstructed epidermis, the composition containing from 0.0005 to 0.05 % by weight of ascorbic acid, from 0.000005 to 0.005 % by weight of 2-O-α-D glucopyranosyl of L-ascorbic acid or 6-O-β-D galactopyranosyl of L-ascorbic acid and 0.000005 to 0.005 % by weight of a metal salt of phosphorylated ascorbic acid relative to the total weight of the composition.

13. The use according to the preceding claim, **characterized in that** said metal salt of phosphorylated ascorbic acid is such as defined in one of claims 3 or 4.
